# EUROPEAN PATENT SPECIFICATION

(11) **EP 1 513 555 B1**
(45) Date of publication and mention of the grant of the patent: **28.03.2007**
(21) Application number: 03729543.3
(22) Date of filing: 14.01.2003
(51) Int. Cl.: A61K 47/02, A61P 9/14

(54) **FORMULATIONS OF QUINAPRIL AND RELATED ACE INHIBITORS**
FORMULIERUNGEN VON QUINAPRIL UND VERWANDTE ACE-HEMMER
FORMULATIONS DE QUINAPRIL ET INHIBITEURS DES ACE CORRESPONDANTS

(30) Priority: 15.01.2002 IS 623302
(43) Date of publication of application: 16.03.2005
(73) Proprietor: Actavis Group hf., 220 Hafnarfjordur (IS)
(72) Inventor: EYJOLFSSON, Reynir, IS-220 Hafnarfjordur (IS)
(74) Representative: Fridriksson, Einar Karl
(86) International application number: PCT/IS2003/000002
(87) International publication number: WO 2003/059388

(56) References cited:
- EP-A- 0 280 999
- WO-A-99/62560

## Description

### FIELD OF THE INVENTION

The present invention is within the field of pharmaceutical formulations of ACE (Angiotensin Converting Enzyme) inhibitors, specifically formulations of quinapril and structurally related compounds.

### TECHNICAL BACKGROUND AND PRIOR ART

Many of the compounds useful as ACE (Angiotensin Converting Enzyme) inhibitors such as as quinapril and structurally related compounds, are prone to degradation. Specifically, such compounds can degrade via (i) cyclization via internal nucleophilic reaction to form substituted diketopiperazines, (ii) hydrolysis of the side-chain ester group, and (iii) oxidation to form products having often unwanted coloration.

Certain stabilizing compositions and formulations of such compounds have been suggested and utilized in the prior art.

EP 317878 suggests coating an active compound of this type with a polymeric protective coating, or mixing the compound with a physiologically tolerated buffer which ensures that a pH in the weakly acid to weakly alkaline range is set up in a pharmaceutical formulation in the presence of moisture, or both.

The Dictionnaire Vidal (1985) Cahier Complémentaire (p. 10, left col.) describes an enalapril maleate drug named RENITEC which as excipients contains lactose and sodium hydrogen carbonate.

EP 280999B1 suggests similar compositions using alkali or alkaline earth metal carbonates and saccharides as stabilizers for these compounds. Comparative examples therein (see, e.g. Example D) test a formulation with 5.4 mg quinapril hydrochloride, 88.4 mg magnesium carbonate, 5.2 mg gelatin and 1.0 mg magnesium stearate. The formulation, however, shows substantial degradation as compared with lactose-stabilized formulations and is therefore not useful as a practical pharmaceutical formulation.

Said ACE Inhibitor compounds have varied sensitivity and specific formulations need to be tested and optimized for different compounds. Consequently, alternative solutions providing stable formulations will be appreciated.

It has now been surprisingly discovered that useful, stable formulations can be produced with use of excipients comprising a basic compound, preferably an alkali or alkaline-earth metal carbonate, and an insoluble alkaline-earth metal hydrogen phosphate is further used as a preferred filler substance. Surprisingly, a saccharide compound for stabilization is not needed in such formulations.

The pH of the formulations of the present invention is dominated by the basic stabilizer.

Tablets of such formulations have good storage stability, dissolution characteristics, and the formulations are suitable for use in drug combinations.

In one embodiment, 45% of magnesium carbonate is mixed with 3.9% quinapril hydrochloride with the addition of 33% calcium hydrogen phosphate (CaHPO₄). Additional specific embodiments and experimental stability test results are disclosed in the Detailed description below and enclosed Examples.

### DETAILED DESCRIPTION

The pharmaceutical formulation of the present invention comprises 0.5 - 50 wt%, such as 1-25 wt%, including 1-15 wt% of a compound of formula I: 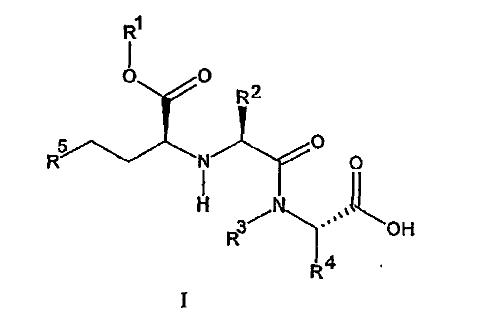 wherein R¹ is hydrogen or alkyl having one to five carbon atoms; R² is hydrogen or C₁-C₄ alkyl or the group 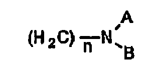 in which A and B independently denote hydrogen or C₁-C₄ alkyl and n is 1-4; R³ and R⁴ together with the atoms they are connected to form a heterocyclic, mono-, di-, or tricyclic ring system which is optionally substituted with C₁-C₄ alkoxy; R⁵ is methyl or phenyl; or any pharmaceutically acceptable salt thereof; 5-90 wt% of an alkali or alkaline earth metal carbonate, such as in the range of 10 - 90 wt%, including the range of about 15-75 wt% and preferably in the range of 25 - 75 wt%, such as 25 - 50 wt% or 30 - 50 wt%; 5-90 wt% including the range of 10 - 90 wt% of an alkali or alkaline earth metal salt of hydrogen phosphate which is preferably an insoluble alkaline-earth metal salt of hydrogen phosphate, such as in the range of 15 - 75 wt%, such as the range of 20 - 60 wt%, and preferably in the range of 25 - 50 wt%, or in the range of 15 - 30 wt%; and containing less than 10 wt% of a saccharide compound.

Preferably the amount of saccharide compound is less than 5 wt%, more preferably less than 2 wt%.

The alkaline earth metal carbonate may suitably be selected from magnesium carbonate, sodium hydrogen carbonate and sodium carbonate.

In preferred embodiments, the amount of the alkaline earth metal carbonate is at least the equivalent of the amount of the active compound of formula 1, such as e.g. at least twice the equivalent, or at least three times the equivalent of the amount of the active compound.

The term equivalent in this context refers to the conventional ionic equivalent term, one equivalent of a substance participating in a neutralization reaction is that amount of a substance that either contributes or consumes 1 mol of hydrogen ions in that reaction. i.e. for a monoacidic compound such as ramipril and a monobasic alkaline compound such as NaHCO₃, the equivalent ratio of the compounds is the same as the molar ratio; for a diacidic compound such as quinapril HCI stabilized with NaHCO₃, one equivalent of NaHCO₃ equals two moles of NaHCO₃; and likewise for a diacidic compound and dibasic allkaline compound such as Na₂CO₃, the equivalent ratio is again the same as the molar ratio. (See, e.g. Skoog, West, Holler *Fundamentals af Analytical Chemistry* 5th Ed., Saunders College Publishing, NY, 1988).

As mentioned, the pH of the formulations are dominated by the basic stabilizing excipient, i.e., the alkali or alkaline-earth metal carbonate.

The ACE inhibitor compound is generally selected from enalapril, delapril, lisinopril, moexipril, perindopril, quinapril, ramipril, trandolapril, and pharmaceutically acceptable salts thereof. In particular, stable formulations of quinapril or a salt thereof are suitably manufactured according to the invention disclosed herein.

In yet further useful embodiments, the formulation of of the present invention further comprises in the range of about 0.5 - 50 wt% of a pharmaceutically active compound selected from the group containing diuretics including hydrochlorothiazide; antitussives including dextromethorphan, dextromethorphan hydrobromide, noscapine, carbetapentane citrate, and chlophedianol hydrochloride; antihistamines including chloepheniramine maleate, phenindamine tartrate, pyrilamine maleate, doxylamine succinate, and phenyltoloxamine citrate; decongestants including phenylephedrine hydrochloride, phenylpropanolamine hydrochloride, pseudoephedrine hydrochloride, ephedrine; and alkaloids such as codeine phosphate, codeine sulfate, and morphine. The suitable amount of a further pharmaceutically active compound such as the above listed depends on the particular compound, i.e. the activity of the compound and its suitable dose, and the dose weight of the pharmaceutical formulation.

### EXAMPLES

### Example 1

The following materials were combined by the wet granulation method for the manufacture of 5 mg quinapril tablets.

| | |
|---|---|
| Quinapril hydrochloride | 5.4 mg |
| Magnesium carbonate | 63 mg |
| Calcium hydrogen phosphate anhydrous | 46.4 mg |
| Starch pregelatinized | 21 mg |
| Croscarmellose sodium | 2.8 mg |
| Magnesium stearate | 1.4 mg |

### Example 2

The following materials were processed by wet granulation for 10/12.5 mg tablets with Quinapril and Hydrochlorothiazide.

| | |
|---|---|
| Quinapril hydrochloride | 10.8 mg |
| Hydrochlorothiazide | 12.5 mg |
| Magnesium carbonate | 49 mg |
| Calcium hydrogen phosphate anhydrous | 42.5 mg |
| Starch pregelatinized | 21 mg |
| Croscarmellose sodium | 2.8 mg |
| Magnesium stearate | 1.4 mg |

### Example 3

Stability of the tablets prepared in the Examples 1-2 were tested at 40°C for 9 days.

| Degradation products (%) | | |
|---|---|---|
| | Quinapril DKP | Quinaprilat |
| Example 1 | 0.2 | 1.2 |
| Example 2 | 0.3 | 0.6 |

| | | |
|---|---|---|
| (DKP = diketopiperazine) | | |

## Claims

1. A pharmaceutical formulation comprising:
a. 0.5 - 50 wt% of a compound of formula I; 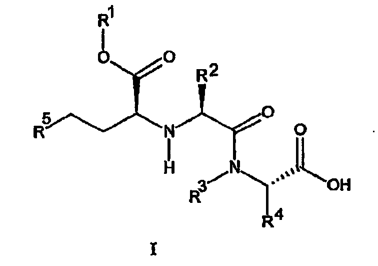 wherein R¹ is hydrogen or alkyl having one to five carbon atoms; R² is hydrogen or C₁-C₄ alkyl or the group 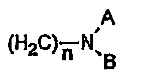 in which A and B independently denote hydrogen or C₁-C₄ alkyl and n is 1-4; R³ and R⁴ together with the atoms they are connected to form a heterocyclic, mono-, di-, or tricyclic ring system which is optionally substituted with C₁-C₄ alkoxy; R⁵ is methyl or phenyl; and pharmaceutically acceptable salts thereof;
b. 5-90 wt% of an alkali or alkaline earth metal carbonate;
c. 5-90 wt% of an insoluble alkaline-earth metal salt of hydrogen phosphate; and
d. containing less than 10 wt% of a saccharide compound.

2. The formulation of claim 1, wherein the alkali or alkaline-earth metal carbonate is selected from magnesium carbonate, sodium hydrogen carbonate and sodium carbonate.

3. The formulation of claim 1, wherein the amount of the alkaline earth metal carbonate is at least the equivalent of the amount of the active compound of formula I.

4. The formulation of claim 1, wherein the compound of formula I is selected from the group containing enalapril, delapril, lisinopril, moexipril, perindopril, quinapril, ramipril, trandolapril, and pharmaceutically acceptable salts thereof.

5. The formulation of claim 1 wherein the formulation comprises in the range of 1 -15 wt% of the compound of formula 1.

6. The formulation of claim 1 wherein the formulation comprises in the range of 25 - 75 wt% of the alkali or alkaline earth metal carbonate.

7. The formulation of claim 6 wherein the formulation comprises in the range of 30 - 50 wt% of the alkali or alkaline earth metal carbonate.

8. The formulation of claim 1 wherein the formulation comprises in the range of 15 - 75 wt% of the salt of an insoluble alkaline earth metal hydrogen phosphate.

9. The formulation of claim 1 wherein the formulation comprises in the range of 25 - 50 wt% of the salt of an insoluble alkaline earth metal hydrogen phosphate.

10. The formulation of any of the preceding claims wherein the compound of formula I is quinapril or a pharmaceutically acceptable salt thereof.

11. The formulation of claim 1 further comprising 0.5 - 50 wt% of a pharmaceutically active compound selected from the group containing diuretics including hydrochlorothiazide; antitussives including dextromethorphan, dextromethorphan hydrobromide, noscapine, carbetapentane citrate, and chlophedianol hydrochloride; antihistamines including chloepheniramine maleate, phenindamine tartrate, pyrilamine maleate, doxylamine succinate, and phenyltoloxamine citrate; decongestants including phenylephedrine hydrochloride, phenylpropanolamine hydrochloride, pseudoephedrine hydrochloride, ephedrine; and alkaloids such as codeine phosphate, codeine sulfate, and morphine.

## Patentansprüche

1. Pharmazeutische Formulierung, umfassend:
a. 0,5 - 50 Gew. -% einer Verbindung von Formel I: 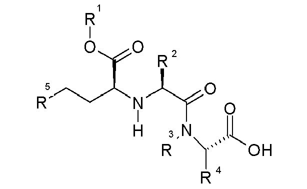 wobei R¹ ein Wasserstoff oder Alkyl mit einem bis fünf Kohlenstoffatomen ist; R² Wasserstoff oder C₁-C₄-Alkyl oder die Gruppe 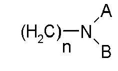 ist, in der A und B unabhängig Wasserstoff oder C₁-C₄-Alkyl bezeichnen und n 1-4 ist; R³ und R⁴ zusammen mit den Atomen, mit denen sie verbunden sind, ein heterozyklisches, mono-, di- oder trizyklisches Ringsystem bilden, welches gegebenenfalls mit C₁-C₄-Alkoxy substituiert ist; R⁵ Methyl oder Phenyl ist, und pharmazeutisch annehmbare Salze davon.
b. 5 - 90 Gew.-% eines Alkali- oder Alkalierdmetallcarbonats;
c. 5 - 90 Gew.-% eines unlöslichen Alkalierdmetallsalzes von Hydrogenphosphat; und
d. enthaltend weniger als 10 Gew.-% einer Saccharidverbindung.

2. Formulierung nach Anspruch 1, wobei das Alkali- oder Alkalierdmetallcarbonat aus Magnesiumcarbonat, Natriumhydrogencarbonat und Natriumcarbonat ausgewählt ist.

3. Formulierung nach Anspruch 1, wobei die Menge des Alkalierdmetallcarbonats mindestens dem Äquivalent der Menge der aktiven Verbindung von Formel I entspricht.

4. Formulierung nach Anspruch 1, wobei die Verbindung von Formel I aus der Gruppe ausgewählt ist, die Enalapril, Delapril, Lisinopril, Moexipril, Perindopril, Qulnapril, Ramipril, Trandolapril und pharmazeutisch annehmbare Salzen davon enthält.

5. Formulierung nach Anspruch 1, wobei die Formulierung im Bereich von 1 - 15 Gew.-% der Verbindung von Formel I aufweist.

6. Formulierung nach Anspruch 1, wobei die Formulierung im Bereich von 25 - 75 Gew.-% des Alkali- oder Alkalierdmetallcarbonats aufweist.

7. Formulierung nach Anspruch 6, wobei die Formulierung im Bereich von 30 - 50 Gew.-% des Alkali- oder Alkalierdmetallcarbonats aufweist.

8. Formulierung nach Anspruch 1, wobei die Formulierung im Bereich von 15 - 75 Gew.-% des Salzes eines unlöslichen Alkalierdmetallhydrogenphosphats aufweist.

9. Formulierung nach Anspruch 1, wobei die Formulierung im Bereich von 25 - 50 Gew.-% des Salzes eines unlöslichen Alkalierdmetallhydrogenphosphats aufweist.

10. Formulierung nach einem der vorherigen Ansprüche, wobei die Verbindung von Formel I Qulnapril oder ein pharmazeutisch annehmbares Salz davon ist.

11. Formulierung nach Anspruch 1, des Weiteren umfassend 0,5 - 50 Gew.-% einer pharmazeutisch aktiven Verbindung, die aus der Gruppe ausgewählt ist, die Diuretika, einschließlich Hydrochlorthiazid, Antitussiva, einschließlich Dextromethorphan, Dextromethorphanhydrobromid, Noscapin, Carbetapentancitrat und Chlophedianolhydrochlorid, Antihistamine, einschließlich Chloepheniraminmaleat, Phenindamintartrat, Pyrilaminmaleat, Doxylaminsuccinat und Phenyltoloxamincitrat, Dekongestiva, einschließlich Phenylephedrinhydrochlorid, Phenylpropanolaminhydrochlorid, Pseudoephedrinhydrochlorid, Ephedrin, und Alkaloide wie beispielsweise Codeinphosphat, Codeinsulfat und Morphin enthält.

## Revendications

1. Formulation pharmaceutique comprenant :
a. de 0,5 à 50 % en poids d'un composé de formule I ; 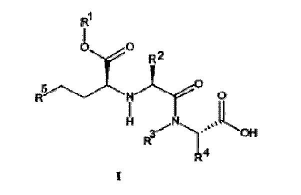 dans laquelle R¹ est un atome d' hydrogène ou un groupe alkyle ayant de 1 à 5 atomes de carbone ; R² est un atome d'hydrogène ou un groupe alkyle en C₁-C₄ ou le groupe 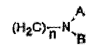 dans lequel A et B représentent indépendamment un atome d'hydrogène ou un groupe alkyle en C₁-C₄ et n est 1 à 4 ; R³ et R⁴ conjointement avec les atomes auxquels ils sont liés forment un système cyclique hétérocyclique, mono-, di-, ou tricyclique qui est facultativement substitué par un groupe alcoxy en C₁-C₄; R⁵ est un groupe méthyle ou phényle ; et les sels pharmaceutiquement acceptables de celui-ci ;
b. de 5 à 90% % en poids d'un carbonate de métal alcalin ou alcalino-terreux;
c. de 5 à 90% en poids d'un sel d'hydrogénophosphate de métal alcalino-terreux insoluble ; et
d. contenant moins de 10 % en poids d'un composé saccharide.

2. Formulation selon la revendication 1, dans laquelle le carbonate de métal alcalin ou alcalino-terreux est choisi parmi le carbonate de magnésium, l'hydrogénocarbonate de sodium et le carbonate de sodium.

3. Formulation selon la revendication 1, dans laquelle la quantité de carbonate de métal alcalino-terreux est au moins l'équivalent de la quantité du composé actif de formule I.

4. Formulation selon la revendication 1, dans laquelle le composé de formule I est choisi dans le groupe contenant l'énalapril, le délapril, le lisinopril, le moexipril, le perindopril, le quinapril, le ramipril, le trandolapril, et les sels pharmaceutiquement acceptables de ceux-ci.

5. Formulation selon la revendication 1, dans laquelle la formulation comprend le composé de formule 1 dans la plage de 1 à 15 % en poids.

6. Formulation selon la revendication 1, dans laquelle la formulation comprend le carbonate de métal alcalin ou alcalino-terreux dans la plage de 25 à 75 % en poids.

7. Formulation selon la revendication 6, dans laquelle la formulation comprend le carbonate de métal alcalin ou alcalino-terreux dans la plage de 30 à 50 % en poids.

8. Formulation selon la revendication 1, dans laquelle la formulation comprend le sel d'un hydrogénophosphate de métal alcalino-terreux insoluble dans la plage de 15 à 75 % en poids.

9. Formulation selon la revendication 1, dans laquelle la formulation comprend le sel d'un hydrogénophosphate de métal alcalino-terreux insoluble dans la plage de 25 à 50% % en poids.

10. Formulation selon l'une quelconque des revendications précédentes, dans laquelle le composé de formule I est le quinapril ou un sel pharmaceutiquement acceptable de celui-ci.

11. Formulation selon la revendication 1, comprenant en outre de 0,5 à 50% en poids d'un composé pharmaceutiquement actif choisi dans le groupe contenant des diurétiques comprenant l'hydrochlorothiazide ; des antitussifs comprenant le dextrométhorphane, le bromhydrate de dextrométhorphane, la noscapine, le citrate de carbétapentane, et le chlorhydrate de chlophédianol ; des antihistamines comprenant le maléate de chlorphéniramine, le tartrate de phénindamine, le maléate de pyrilamine, le succinate de doxylamine, et le citrate de phényltoloxamine; des décongestionnants comprenant le chlorhydrate de phényléphédrine, le chlorhydrate de phénylpropanolamine, le chlorhydrate de pseudoéphédrine, l'éphédrine ; et des alcaloïdes tels que le phosphate de codéine, le sulfate de codéine, et la morphine.
